# EUROPEAN PATENT SPECIFICATION

(11) **EP 0 769 048 B1**
(45) Date of publication and mention of the grant of the patent: **06.04.2005**
(21) Application number: 95924749.5
(22) Date of filing: 27.06.1995
(51) Int. Cl.: C12N 5/02, C12M 3/06

(54) **COMPARTMENTALIZED TISSUE CULTURE FLASK**
ZELLKULTURFLASCHE MIT MEHREREN KAMMERN
RECIPIENT DE CULTURE TISSULAIRE A COMPARTIMENTS

(30) Priority: 28.06.1994 US 268073; 18.10.1994 US 324563; 30.03.1995 US 413334
(43) Date of publication of application: 23.04.1997
(73) Proprietor: Wilson Wolf Corporation, New Brighton, MN 55112 (US)
(72) Inventor: Wilson, John, New Brighton, MN 55112 (US); Wolf, Martin L., Saint Paul, MN 55108 (US)
(74) Representative: Jany, Peter, Dr.
(86) International application number: PCT/US1995/008202
(87) International publication number: WO 1996/000780

(56) References cited:
- EP-A- 0 155 237
- EP-A- 0 307 048
- WO-A-90/05179
- US-A- 4 661 455
- US-A- 4 748 124
- US-A- 4 937 196
- GIBCO BRL CATALOGUE & REFERENCE GUIDE, Published 1992, by LIFE TECHNOLOGIES, INC., pages 6-8 and 78.

## Description

### BACKGROUND - FIELD OF THE INVENTION

This invention relates to a device and a method for growing cells or tissue In vitro.

### BACKGROUND - DESCRIPTION OF PRIOR ART

In vitro growth of mammalian cells is commonly conducted in static culture vessels such as tissue culture flasks (U.S. Pat. No. 3,449,210 issued June 10, 1969 and U.S. Pat. No. 5,151,366 issued Sept. 29, 1992), spinner flasks, and multiple well plate tissue culture plates (U.S. Pat. No. 3,597,326 issued Aug. 3, 1971 and U.S. Pat. No. 4,012,288 issued March 15, 1977). In this type of culture, a portion of the cell culture medium is periodically removed and replaced as cells consume nutrients and produce waste products. This protocol leads to limited cell density, limited cell secreted product concentration, and periodic shifts in nutrient concentration.

Marbrook used a dialysis membrane to separate cells and cell secreted products from the basal medium (Marbrook, J., "Primary Immune Response in Cultures of Spleen Cells", the Lancet, 2, 1279-1281 [1967]). In this device, an inner concentric chamber resides within an outer concentric chamber. The bottom of the inner chamber is comprised of a dialysis membrane which is submerged in basal medium contained in the outer chamber. Cells reside on the membrane receiving nutrients and delivering waste products. Continuous dialysis becomes limited as the membrane loses substrate transport capacity due to the cell mass that resides upon it. Thus, the ability to carry out long term culture is compromised.

Verma (U.S. Patent No. 4,296,205 issued October 20, 1981) teaches of the use of a tissue culture shelf placed in the cell culture compartment to keep cells from directly contacting and clogging the dialysis membrane. The tissue culture shelf has perforations to allow movement of nutrients to the cells. During the culture of suspension cells, the cells and cellular debris are capable of moving through the perforations and coming to rest upon the dialysis membrane, limiting continuous dialysis in long term culture. Also, the architectural structure of the shelf can lead to microenvironments as concentration gradients are unevenly distributed across the surface of the plate.

Vogler (U.S. Patent No. 4,748,124 issued May 31,1988) describes a cell culture compartment that is defined by a lower gas permeable, liquid impermeable sheet and an upper dialysis membrane. This configuration keeps the dialysis membrane from clogging as cells do not reside upon it, yet dialysis can become limited by other means. Also, the ability to vary oxygen tension is limited relative to Marbrook and Verma. Furthermore, the surface chemistry of materials used to allow gas transfer are limited and in some cases can be undesirable for protein or cell contact. Finally, the teaching does not lead to high density cell culture relative to traditional static culture methods.

The architecture of Vogler can allow dialysis of the cell compartment to become limited. A major problem can arise as liquid evaporates from the growth chamber. Vapor transmission across gas permeable surfaces can be substantial and the loss of liquid will lead to termination of dialysis as liquid contact with the dialysis membrane ceases. Loss of dialysis will also result from off gassing of cell culture medium. Cell culture medium is typically stored at 4 degrees Celsius. As the medium rises in temperature, the gas carrying capacity is reduced and gas bubbles rise and come in contact with the dialysis membrane.

The gas permeable, liquid impermeable sheet of the cell culture compartment limits options available for controlling pericellular pH and P_{O2}. In the prior configurations of Marbrook and Verma, the oxygen tension could be varied by adjusting the liquid level of the cell culture compartment. The structure and method taught by Vogler require oxygen tension be varied by altering the ambient conditions of the atmosphere surrounding the device.

Oxygen tension is very important to cell viability and protein secretion (Reuveny et al., "Factors Affecting Cell Growth and Monoclonal Antibody Production in Stirred Reactors", Journal of Immunological Methods, 86, 53-59 [1986]). The gas permeability of commercially available liquid impermeable sheets and the impact upon pericellular pH and P_{O2} is described in detail by Jenson et al. (Jenson M.D., Wallach D.F.H., and Sherwood P., "Diffusion in Tissue Cultures on Gas- permeable and Impermeable Supports", J. Theor. Biol. (1976) 56, 443-458). The oxygen demands of various cell types combined with the gas permeability of various gas permeable, liquid impermeable sheets will dictate a specific steady state pericellular pH and P_{O2} for each combination. This means cell lines are subject to very limited pericellular conditions. Creating different pericellular conditions is achieved by altering the ambient conditions of the incubator in which the device resides. As a practical matter, this is difficult for researchers who maintain incubators at standard conditions for a wide variety of simultaneous uses.

Gas permeable, liquid impermeable sheets also limit the surface chemistry available for support of cells and protein structures. The proliferation and function of many cell types is strongly affected by the chemical nature of the surfaces they reside upon. The surface chemistry of liquid impermeable material is incompatible with many cell types and protein structures. Also, hydrophobic material which is often the basis for gas permeable, liquid impermeable films, can cause non-specific protein binding. This in turn can lead to depletion of soluble growth factors. Thus, further modification to the materials may be required for optimization of the cell environment.

The architecture of Vogler also leads to limited cell density. The growth chamber will deform in shape due to the weight of liquid residing upon it and pressure of fluid expansion, leading to a sagging gas permeable sheet. This allows suspension cells to settle in the low point of the sheet. High localized cell densities at the low point of the sheet leads to excessive resistance to flux of nutrients and a localized reduction in cell viability. Furthermore, the cells are unable to expand to other areas of the gas permeable sheet.

Hubbard (US 4,661,455) provides an apparatus for culturing cells comprising a first chamber containing oxygen, a second chamber containing cells and a third chamber containing nutrients. The apparatus comprises a plastic housing containing a double membrane. This reference teaches the need for two ports (one for fluid addition and one for fluid removal) into the cell compartment. A pumping mechanism is used to control flow into and out of the basal medium and cell compartments upon a perfusion method. This apparatus is not sufficiently simple for research and mid-scale culture applications.

Wrasidlo (US 4,937,196) discloses a complex bioreactor in which cells are confined to a compartment formed by sheet membranes. The device integrates the components to grow cells and to extract concentrated cell secreted products. Gas compartments allow the flow of free oxygen into the cell compartment. This reference teaches the need for two ports (one for fluid addition and one for fluid removal) into the cell compartment. A pumping mechanism is used to control flow into and out of the basal medium and cell compartments. This apparatus is not sufficiently simple for research and mid-scale culture applications.

Katinger (EP 0155237 A2) is directed to an apparatus for culturing cells having separate cell growth, medium and production chambers which are separated by membranes. A nutrient solution flows through the medium chamber wherein the contents of the other chambers are stationary. This reference teaches the need for two ports (one for fluid addition and one for fluid removal) into the cell compartment, wherein perfusion is involved for medium circulation. Gas supply and removal is performed by controlling the gas concentration of the medium residing in an external container. This apparatus is not sufficiently simple for research and mid-scale culture applications.

Cremonese (EP 0307048 A2) discusses a cell culture flask including a hollow housing and a gas permeable membrane disposed within the housing which divides the housing into two chambers separated by a membrane. A gas inlet in the housing communicates with the first chamber and a media inlet in the housing communicates with the second chamber. This reference teaches the need for two ports, one for fluid addition and one for fluid removal into the cell compartment, wherein the liquid growth medium is constantly supplied to the cells. Pressurized gas flow is required by way of a gas inlet and a gas outlet. The contamination potential is high.

Howell (WO 90/05179) discloses a perfusable cell culture device capable of expansion and maintenance of biological cells. This device is directed towards large scale cell culture and utilizes a very complex process for optimization that requires customized ancillary equipment. It entirely relies on perfusion (i.e. forced mechanical motion) to function which is a complex and expensive proposition for many laboratories.

It is accordingly an object of the present invention to provide a method and devices for the long term culture of anchorage dependent cells and suspension cells at high density, simultaneously allowing variable oxygen tension, an even distribution of cells across the bottom of the culture compartment, uninterrupted dialysis, and a wide variety of surface chemistry options. Still further objects and advantages will become apparent from consideration of the ensuing description and drawings.

### SUMMARY OF THE INVENTION

Many of the problems of the prior-art are solved by compartmentalized cell culture devices constructed in accordance with this invention to allow cells to be cultured at high density over a long period of time.

Specifically, there is provided a cell culture compartment including a lower gas permeable film and spaced vertically therefrom an upper sheet selectively permeable to compounds of selected sizes, means spacing said film from said sheet so as to allow a culture medium to reside between said upper sheet and said lower gas permeable film. There is a means defining a basal medium compartment to allow a basal medium to reside upon said upper sheet. There are access ports to said cell culture compartment and to said basal medium compartment. There exists a gas film support below and in partial contact with said gas permeable film whereby a major portion of said gas permeable film is held in a substantially horizontal position such that cells can distribute across the horizontal portion of said gas permeable film and gas transfer into and out of said cell culture compartment is not substantially impaired. The gas permeable film can be any biocompatible liquid permeable or impermeable. hydrophobic or hydrophilic, porous or non porous material which provides the appropriate pericellular environment and surface chemistry for a specific call culture application.

The upper basal medium compartment and the lower cell culture compartment are configured to allow pipette access and prevent pressurization due to temperature increase The cell culture compartment is configured to prevent loss of dialysis due to evaporation or off-gassing, compensate for liquid flux from the basal medium reservoir, and allow high cell density cultures to be maintained over a long period of time.

According to one feature of the invention, evaporative loss of cell culture medium can be controlled independent of ambient conditions by providing gaseous exchange of the cell culture compartment by way of the humidified gas of the upper basal medium compartment.

According to a second feature of the invention, oxygen tension within the cell culture compartment can be accurately controlled independent of ambient conditions by adding a third compartment that utilizes a variable level of liquid to alter oxygen tension.

According to a third feature of the invention, the cell culture compartment volume can be varied during operation with out interrupting dialysis.

According to a fourth feature of the invention, the transfer of air to the cell culture compartment can be minimized during routine handling to minimize pH fluctuations

According to another embodiment of the invention, there is provided a plurality of cell culture compartments each including a lower gas permeable film and spaced vertically therefrom an upper sheet selectively permeable to compounds of selected sizes, means spacing said film from said sheet so as to allow a culture medium to reside between said upper sheet and said lower gas permeable film. There is a means defining a basal medium compartment to allow a basal medium to reside upon said upper sheet. There are access ports to each of said cell culture compartments and to said basal medium compartment. There exists a gas film support below and in partial contact with each of said gas permeable films whereby a major portion of each gas permeable film is held in a substantially horizontal position such that suspension cells can distribute across the horizontal portion of said gas permeable film and gas transfer into and out of said cell culture compartment is not substantially impaired.

With these structures, a method of culturing cells at high density becomes available. Also, a method of controlling oxygen tension surrounding cells becomes available by utilizing a liquid barrier to oxygen flux.

With the invention so stated, many of the problems associated with the prior art are solved. Long term high density in vitro culture of both suspension and adherent cells is possible with simultaneous provisions for variable oxygen tension, controlled evaporation, long term maintenance of small cell compartment liquid volumes, and uninterrupted dialysis.

### A BRIFF DESCRIPTION OF THE DRAWINGS

Fig. 1 is a cut away view taken through the center of a compartmentalized tissue culture flask;
Fig. 2 is a cross-sectional view of a compartmentalized tissue culture flask showing an embodiment using a gas permeable membrane, portions of which project into the cell culture compartment.
Fig. 3 is a cross-sectional view of a compartmentalized tissue culture flask showing an embodiment that balances hydrostatic pressure in two compartments.
Fig. 4 is a cross-sectional view of a compartmentalized tissue culture flask showing an embodiment that controls evaporation; and
Fig. 5 is a cutaway view of a compartmentalized flask that minimizes pH fluctuations during routine handling.
Fig. 6 is a side view of a compartmentalized flask standing on end.
Fig. 7 is a cross-sectional view of compartmentalized tissue culture flask showing an embodiment that allows variable oxygen tension.
Fig. 8 is a top view of a compartmentalized multiple well tissue culture plate in accordance with the present invention;
Fig. 9 is a partial cross-section taken through section A-A of the multiple well tissue culture plate of Fig. 8; and

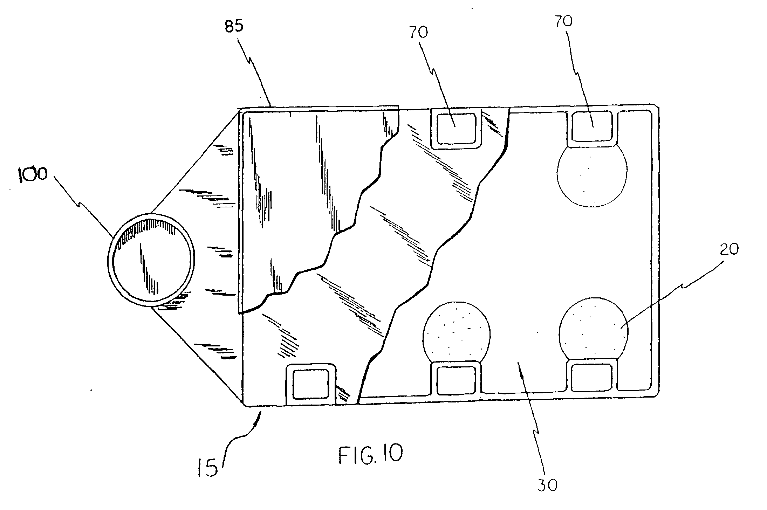

Fig. 10 is a top view of a compartmentalized tissue culture plate in which basal medium resides in a common reservoir.

### Reference Numerals in Drawings

- 10: compartmentalized tissue culture flask
- 15: compartmentalized multiple well tissue culture plate
- 20: membrane
- 30: basal medium compartment
- 40: cell culture compartment
- 50: culture medium
- 60: basal medium
- 70: cell culture compartment access port
- 80: cell culture compartment access port cap
- 85: top cover
- 90: basal medium access port
- 100: basal medium access port cap
- 110: membrane support
- 120: gas permeable film
- 130: gas film support
- 140: gas access opening
- 150: feet
- 170: basal medium head space
- 180: culture medium head space
- 190: gas access channel
- 200: gas access channel cover
- 210: drain port
- 220: variable oxygen ,control compartment
- 230: lower gas permeable film
- 240: oxygen control compartment bottom
- 250: oxygen control compartment access port
- 260: liquid resistor
- 270: upper membrane support
- 280: skirt
- 290: notches
- 300: notch cover
- 310: notch cover hinge

### DETAILED DESCRIPTION

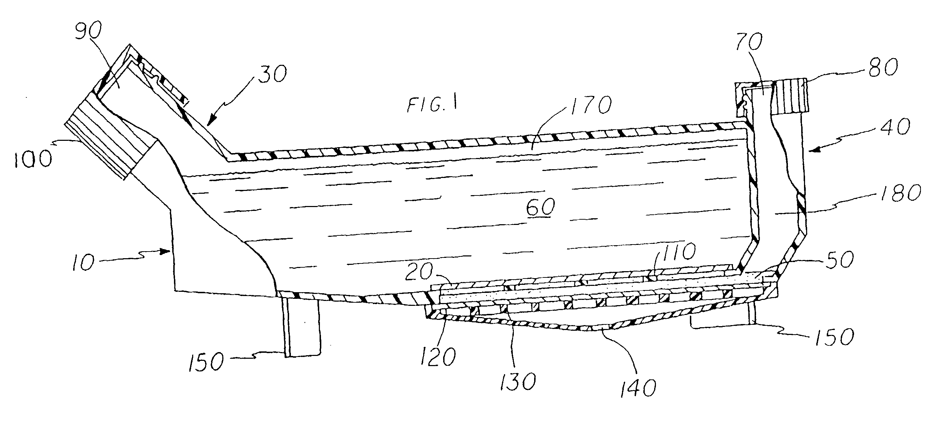

Referring now more specifically to the drawings, Fig. 1 shows a cutaway view of the invention in the embodiment of a compartmentalized tissue culture flask 10. A membrane 20 separates compartmentalized tissue culture flask 10 into a basal medium compartment 30 and a cell culture compartment 40. A culture medium 50 containing cells or tissue resides in cell culture compartment 40. A basal medium 60 resides in basal medium compartment 30. Access to cell culture compartment 40 is provided by a cell culture compartment access port 70 which is covered by a cell culture compartment access port cap 80. Access to basal medium compartment 30 is provided by a basal medium access port 90 which is covered by a basal medium access port cap 100. A membrane support 110 stabilizes membrane 20. A gas permeable film 120 resides on top of a gas film support 130 which is adapted to allow gas to contact the vast majority of the surface of gas permeable film 120 by way of a gas access opening 140. Feet 150 lift gas film support 130 above the surface on which compartmentalized tissue culture flask 10 resides.

In operation, culture medium 50 containing cells or tissue of interest is introduced into cell culture compartment 40 through cell culture compartment access port 70 until it makes complete contact with the underside of membrane 20. Basal medium 60 is introduced into basal medium compartment 30 through basal medium access port 90. Preferably, a basal medium head space 170 will be maintained between basal medium 60 and the top of basal medium compartment 30 and basal medium access port cap 100 will be slightly loosened. This allows ambient gas to influence the pH of basal medium 60. A basal medium access port cap of the type used in Falcon® tissue culture flasks (commercially available from Becton Dickinson Labware - Plymouth, England) may be used in cases where the cap should remain tightened due to contamination concerns.

Compartmentalized tissue culture flask 10 is designed to prevent pressurization as the temperature of the gas and liquid it contains rises when it is placed into an incubator. Cell culture access port cap 80 and basal medium access port cap 100 can be loosened to allow expanding gas to vent to the surrounding atmosphere Culture medium 50 is free to expand into a culture medium head space 180 and basal medium 60 is free to expand into basal medium head space 170. In this manner pressure does not affect the flatness of gas permeable film 120, or liquid flux through either membrane 20 or gas permeable film 120.

Aside from fluid expansion, another phenomenon occurs as a result of the temperature increase. The gas carrying capacity of the liquid medium is lowered. Gas bubbles that are released by culture medium 50 can be moved to culture medium head space 180 by temporarily tilting compartmentalized tissue culture flask 10. This prevents the gas from becoming trapped against the bottom of membrane 20 and limiting dialysis.

Culture medium head space 180 is designed to counterbalance liquid transfer that may occur through membrane 50 due to hydrostatic pressure differential that results from head height differences in basal medium 60 and culture medium 50 at the time of set up. As liquid moves through membrane 20 into cell culture compartment 40, the level of culture medium in head space 180 rises. If cell culture compartment access port cap 80 is in the tightened position, the liquid will continue to rise in culture medium head space 180 until the hydrostatic pressure of culture medium 50 is balanced by the pressure of the displaced gas. If cell culture compartment access port cap 80 is in the loosened position, liquid will rise in culture medium head space 180 until it reaches a level where the diminished pressure differential across membrane 50 stops liquid transfer. In the preferred embodiment, the volume of culture medium 50 after flow has stopped will be no more than two times the volume of culture medium 50 at the onset of the culture.

Membrane 20 is a material selectively permeable to a class of molecules. Several types of material are acceptable including cellulose, polyacrylinitrile, polysulfone, polycarbonate, and polyacrilamide. For example, dialysis membranes retaining molecules and compounds with molecular weights greater than 15.000 are commonly used to culture murine hybridoma cells. By using a membrane with this characteristic, cells, growth factors, and secreted antibodies are retained in cell culture compartment 40. In other applications, it may be advantageous to allow larger molecules and compounds to pass freely between basal medium 60 and culture medium 50. For example, high density culture of lymphocytes may require a large quantity of growth stimulating factors to be present. These factors, such as interleukin 2, can be introduced into basal medium 60 and culture medium 50. By appropriately selecting the pore size of membrane 20, a large source of these factors can be made available to the lymphocytes.

Membrane 20 will not exceed a molecular weight cutoff of 150,000 Daltons in most applications. Yet, there are applications where even larger pore sizes may be desirable. For example, if the purpose is only to culture a large number of cells, any pore size which retains the cells in cell culture compartment 40 can be used. In this case, a 0.2uM or 0.45uM microporous polycarbonate membrane such as that commercially available from Poretics Corporation (Livermore, California) could be used.

Membrane support 110 stabilizes membrane 20. As basal medium 60 is added to basal medium compartment 30, the weight is transferred to membrane 20. Membrane support 110 keeps membrane 20 from sagging and displacing culture medium 50 into culture medium head space 180. Membrane support 110 makes minimal contact with membrane 50 so the surface area for dialysis is not substantially diminished. Membrane support 110 is designed such that it will allow gas bubbles to move freely to culture medium head space 180. Membrane support 110 can be made of any biocompatible material. In the preferred embodiment it is clear plastic such as polystyrene or polycarbonate. If membrane 20 is a material cast onto a stiff mesh backing or precise control of the volume of culture medium 50 residing above gas permeable film 120 is not required, membrane support 110 is optional.

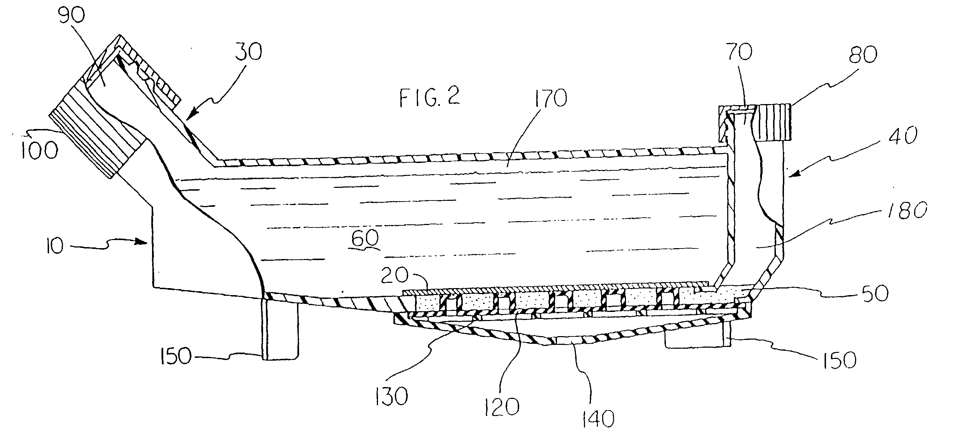

The embodiment of Fig. 2 shows a configuration where portions of gas permeable film 120 project into cell culture compartment 40 to provide support of membrane 20 without the need for membrane support 110. Silicone is a good choice for material as it can be readily molded to form an appropriate shape. Wall thickness can be minimized to allow additional gas transfer into cell culture compartment 40. In the case of silicone, average wall thickness should be kept below 0.015 inches, preferably about 0.004 to 0.012 inches.

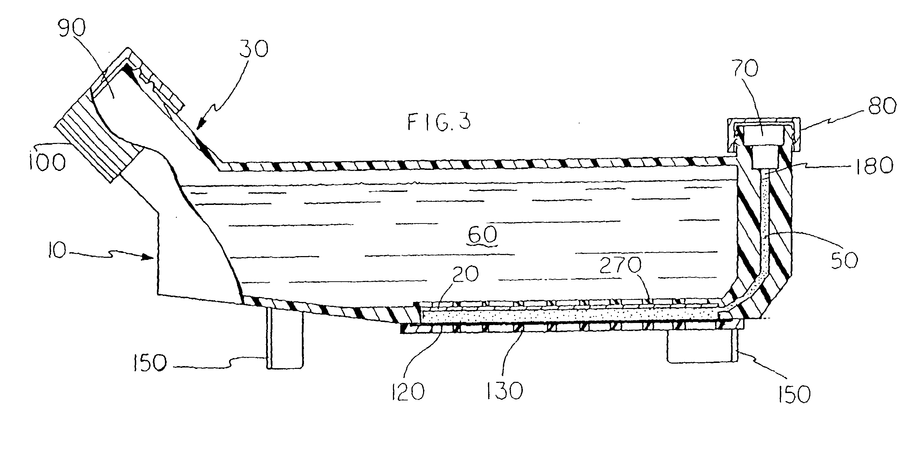

The embodiment shown in Fig. 3 keeps membrane 20 from sagging and insures liquid maintains contact with the upper and lower surface of membrane 20 during operation. It is particularly useful for applications in which a high concentration of cells is desired. Membrane support 110 is not present and this allows a very small volume of culture medium 50 to be used as well as preventing obstacles to cell removal. This embodiment is also capable of functioning with various volumes of culture medium 50, as liquid contact with membrane 20 is always assured.

In operation, membrane 20 is pressed onto the surface of gas permeable film 120 by the weight of basal medium 60. Putting the membrane in this position can also be achieved by generating a vacuum on cell culture compartment 40. A predetermined volume of culture medium 50 containing the desired culture is then introduced into cell culture compartment 40 by way of cell culture compartment access port 70. When basal medium access port cap 100 and cell culture compartment access port cap 80 are in the vented position, culture medium 50 will come to rest within culture medium head space 180 at a level that counterbalances the hydrostatic pressure of basal medium 60.

In the preferred embodiment, the volume of culture medium 50 residing in culture medium head space 180 will be a small fraction of the volume of culture medium 50 residing between membrane 20 and gas permeable film 120. It is possible for water from basal medium 50 to move into cell culture compartment 40 if severe osmotic gradients develop across membrane 20. If this condition begins to occur, cell culture compartment access port cap 80 should be placed in the tightened position. This will prevent liquid from rising in culture medium head space 180.

Introducing culture medium 50 into cell culture compartment 40 will require enough pressure to overcome the hydrostatic pressure of basal medium 60. This can be accomplished by configuring cell culture compartment assess port 70 to accept a pipette, syringe, or some other culture medium container such as a bag or bottle. Culturc medium 50 can be removed in the same manner.

This method of introducing culture medium 50 into cell culture compartment 40 and removing it therefrom can be utilized in all of the embodiments described herein. In most embodiments using membrane support 110, it will be necessary to provide a vent to allow air access to and from cell culture compartment 40. The vent is required to allow gas to be displaced when culture medium 50 is introduced into culture compartment 40. The vent also allows gas to displace culture medium 50 when it is removed from cell culture compartment 40. Cell culture compartment access port 70 is designed to allow gas to move in and out while culture medium 50 is added and removed. Thus, cell culture compartment 40 is effectively vented.

The embodiment of Fig. 3 does not require a vent. When membrane 20 is pressed against gas permeable film 120, air is displaced from cell Culture compartment 40 prior to introducing culture medium 50. When culture medium 50 is removed, membrane 20 is simply lowered. Thus, there is never the need for gas and liquid to move to and from cell culture compartment 40 simultaneously.

When the embodiment shown in Fig. 3 is used for high density culture, the average distance between membrane 20 and gas permeable film 120 should be less than 5 millimeters, preferably about 1 mm to 2 mm.

The embodiment of Fig. 3 can also be used to prevent evaporation of culture medium 50 from allowing membrane 20 to lose contact with culture medium 50. Membrane 20 is essentially floating on culture medium 50 and as culture medium 50 evaporates through gas permeable film 120, membrane 20 simply gets closer to gas permeable film 120. No dialysis limitation occurs, as membrane 20 is always in contact with culture medium 50.

In cases where membrane 20 is comprised of a material such as cellulose that swells or becomes baggy when wet, it may be desirable to constrain membrane 20 with an upper membrane support 270. Upper membrane support 270 stops upward travel of membrane 20 as culture medium 50 enters cell culture compartment 40. Culture medium 50 presses membrane 20 against upper membrane support 270, smoothing wrinkles.

Wrinkles in membrane 20 lead to an uneven distribution of cells during inoculation. If membrane 20 were severely wrinkled, culture medium 50 would reside within the wrinkles. Then some areas above gas permeable film 120 would have more culture medium 50 residing above it than others. Cells in the inoculum are distributed equally throughout culture medium 50. Eventually, these cells settle onto gas permeable film 120. Area of gas permeable film 120 in which a larger volume of culture medium 50 resides above it will receive more cells. To prevent this condition, the wrinkling of membrane 20 should be minimized.

Upper membrane 270 can be any biocompatible material such as virgin grade polystyrene or polypropylene. Care should be given to insure that it does not limit dialysis. In the preferred embodiment, it should be about 70% to 90% open.

Gas permeable film 120 is a biocompatible material capable of allowing transfer of gas into and out of cell culture compartment 40. Gas permeable film 120 can be either liquid permeable or impermeable, hydrophobic or hydrophilic, porous or non porous. Thickness can range above or below 0.25 mm. The best choice depends on the specific application. As a general guideline, the gas permeability of a given membrane should be considered in addition to the interaction of the membrane with either cells or protein structures. Liquid impermeable films of equivalent thickness will establish various steady state oxygen tension at the cell/film interface. FEP Teflon, silicone, and silicone polycarbonate copolymers will establish higher oxygen tension than polyethylene, polycarbonate, polypropylene, polysulfone, or polypropylene. In applications where protein denaturization, non-specific protein binding, cell membrane damage, or cell attachment is affected by the surface chemistry of the film, hydrophilic surfaces are more suitable. In applications where it is desirable to maintain the entire cell membrane in contact with water, hydrated gels may be most suitable

The use of certain materials not normally associated with gas exchange can expand the options available for controlling oxygen tension at the cell/film interface. For example, non-porous cellulose acetate has a relatively low oxygen gas permeability on the order of 7.3 x 10⁻⁹ cm³•cm1(sec•cm²•atm). When cellulose acetate is made porous, it will increase oxygen permeability as it absorbs culture medium 50 with an oxygen permeability of 1.4 x 10⁻⁶ cm³•cm/(sec•cm²•atm). In this manner, varying oxygen tension can be achieved by controlling the amount of culture medium 50 present in gas permeable film 120. Thus, oxygen tension variations will result by varying either the pore size, porosity, or tortuosity of gas permeable film 120.

Gas film support 130 holds gas permeable film 120 in a substantially horizontal position and stabilizes gas permeable film 120 to prevent sagging. Care should be given to assure the flatness of gas permeable film is such that cells do not roll into or otherwise collect in low points. This is an undesirable event as the piling up of cells will create diffusional limitations and lead to cell death. On the other hand, care must also be taken to assure that gas exchange remains adequate. Thus, the optimal amount of contact gas film support 130 makes with gas permeable film 120 will depend on the stiffness and gas permeability of gas permeable film 120 as well as gas exchange and metabolic requirements of a particular cell culture application. It should be expected that most cell lines will become diffusionally limited at about ten to fifteen cell layers.

Gas film support 130 also acts to protect gas permeable film 120 from contamination or puncture. Minimal contact with gas permeable film 120 is made to allow the maximum possible surface area for gas transfer. Gas access opening 140 is located at the lowest point of gas film support 130 to allow condensation to exit gas film support 130. It is sized to allow adequate gas exchange of cell culture compartment 40 while minimizing evaporation. Gas film support 130 can be made of any structurally stable material, but in the preferred embodiment is an optically clear material such as polystyrene or polycarbonate to allow visual inspection of the culture in cases where gas permeable film 120 is optically clear. Feet 150 elevate compartmentalized tissue culture flask 10 such that gas film support 130 does not become scratched or visually impaired.

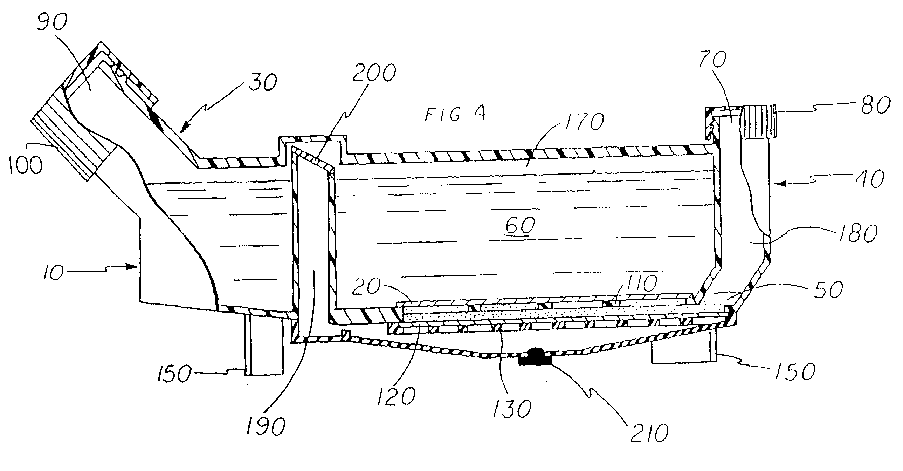

Another consideration with regard to material selection for gas permeable film 120 is the moisture vapor transmission rate. Culture medium 50 will evaporate at various rates pending the material selection of gas permeable film 120. Limiting the cross-sectional area of gas access opening 140 can reduce the rate of evaporation, although the rate of liquid loss will also be a function of the ambient humidity which is more difficult to control. The configuration of Fig. 4 addresses this issue.

The humidified gas of basal medium head space 170 is placed in communication with the underside of gas permeable film 120 by a gas access channel 190. A gas access channel cover 200 prevents basal medium 60 from entering gas access channel 190 and limiting gas transfer. Gas access channel cover 200 is a gas permeable, liquid impermeable film. To prevent condensation from accumulating upon gas access channel cover 200 and diminishing gas transfer, it is not in a horizontal position. Thus condensation can return to basal medium 60 by gravitational force. Also, gas access channel 190 is capable of collecting condensation in a drain port 210.

Many other methods of placing basal medium head space 170 in communication with gas permeable film 120 are possible. Care should be given to prevent condensation or basal medium 60 from diminishing gas transfer.

The configuration of Fig. 4 can also assist in pH control when the device is removed from the incubator for routine handling. Normally, compartmentalized tissue culture flask 10 resides in a CO₂ incubator. This allows the pericellular pH of cell culture compartment 40 to be maintained at an appropriate level. When compartmentalized tissue culture flask 10 is removed from an incubator and placed into a laminar flow hood for cell handling, the pH of cell culture compartment 40 can fluctuate rapidly as CO₂ is stripped from culture medium 50. The configuration of Fig. 4 allows the CO₂ of basal medium compartment 30 to become accessible to culture medium 50

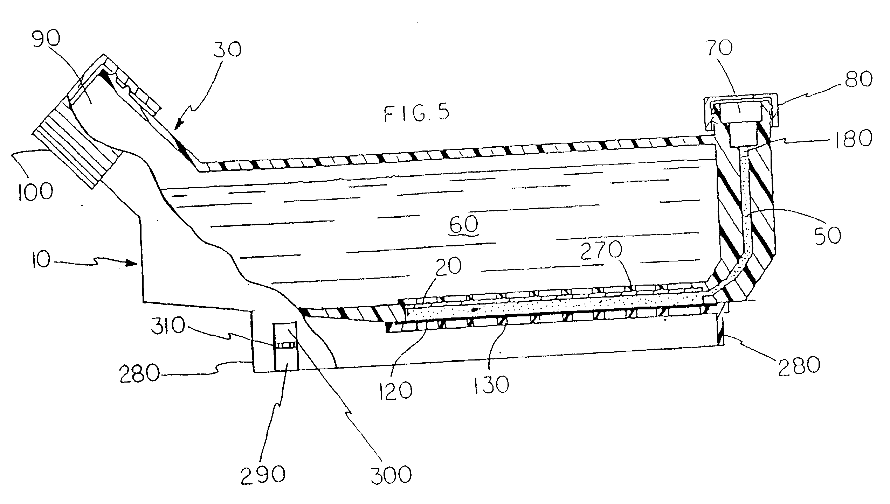

Another configuration that addresses this issue is shown in Fig. 5. A skirt 280 surrounds the base of compartmentalized tissue culture flask 10. Skirt 280 severely restricts air access to the underside of gas permeable film 120 when the device resides in a laminar flow hood. Thus, CO₂ is retained in culture medium 50, maintaining the pH level. The shelves of most CO₂ incubators have openings that will allow gas direct access to the underside of gas film support 130. Thus, skirt 280 does not impede gas transfer when the device resides in most incubators. For the case where the incubator does not have perforated shelves, notches 290 can be created in skirt 280. A notch cover 300 covers each of notches 290. Notch cover 300 rotates on notch cover hinge 310 and is placed in the closed position when the device is removed from the incubator and in the open position when the device resides in the incubator. Fig. 5 shows notch cover in the open position. Many other mechanisms for allowing the skirt to be either open to gas access or closed to gas access are possible.

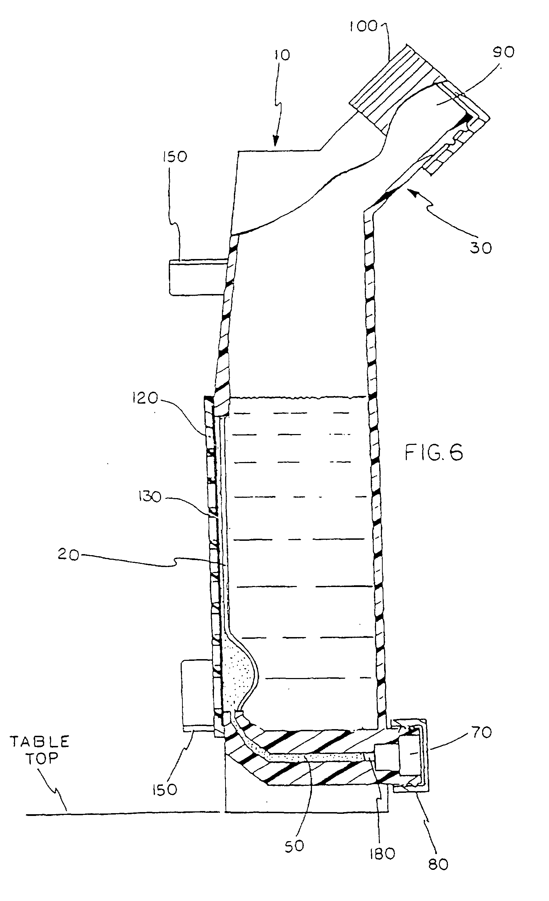

Fig. 6 shows another configuration of compartmentalized tissue culture flask 10 which also limits gas transfer when the device is removed from the incubator. In this configuration, the device stands on end. Culture medium 50 collects in the lower portion of cell culture compartment 40. The contact area between culture medium 50 and gas permeable film 120 is significantly reduced. Thus, gas transfer is reduced in direct proportion to the reduced contact area. This reduces the movement of CO₂ from culture medium 50 to the ambient environment and delays a shift in pH.

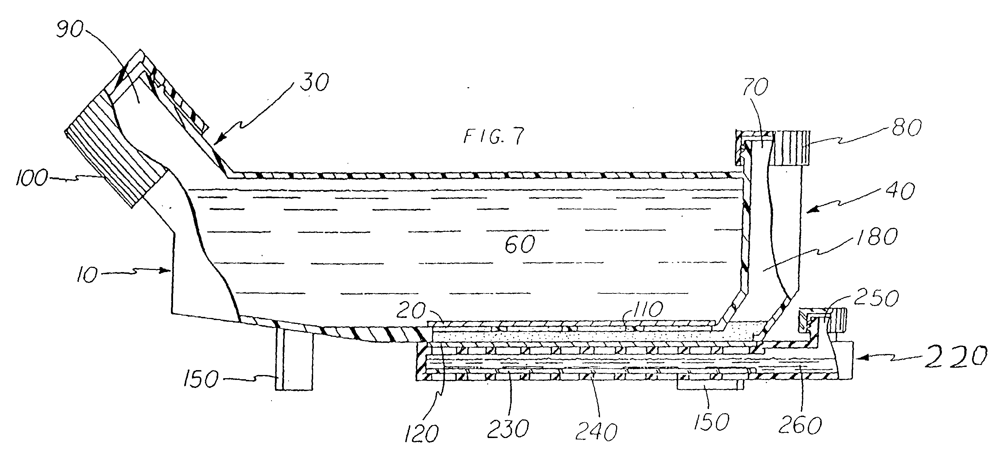

If the type of materials available for gas permeable fiim 120 do not provide the desired oxygen tension, the configuration shown in Fig. 7 can be utilized. A variable oxygen control compartment 220 is composed of a lower gas permeable film 230 supported in a horizontal position by an oxygen control compartment bottom 240. An oxygen control compartment access port 250 allows a liquid resistor 260 to be introduced into variable oxygen control compartment 220. The oxygen tension at the bottom of gas permeable film 120 can be carefully controlled by varying the height of liquid residing upon a lower gas permeable film 230 in accordance with Fick's Law. Lower gas permeable film 230 can be any highly gas permeable film or sheet. In the preferred embodiment, it is liquid impermeable and has a relatively low moisture vapor transmission rate. Oxygen control compartment bottom 240 allows the vast majority of lower gas permeable film 230 to be in communication with the ambient environment. A hermetic seal exists between lower gas permeable film 230 and oxygen control compartment bottom 240. This seal can be made by welding, adhesives, or any other suitable method. The distance between the top of lower gas permeable film 230 and the bottom of gas permeable film 120 will preferably be about 5 to 20 mm.

To minimize evaporation of liquid residing in variable oxygen control compartment 220, the underside of lower gas permeable film 230 can be placed in gaseous communication with basal medium head space 170 as previously described.

Although there is no restriction on either the shape or size of cell culture compartment 40, the advantageous distance between gas permeable film 120 and membrane 20 is about 1 to 5 millimeters to obtain a high concentration of cells and cell secreted products. When gas permeable film 120 is substantially flat and horizontal, up to 30 x 10⁶ cells per square centimeter of surface area can be expected to remain viable. These cells can pile up to a height of about 300 micrometers. Thus, membrane 20 is in no danger of contacting cells and becoming clogged when it resides at least 1 mm from gas permeable film 120.

In order to minimize the frequency of basal medium 60 exchanges, the volume of basal medium 30 is sized relative to the surface area of gas permeable film 120. For suspension cells that reside in static culture at one million cells per milliliter, about 5 to 10 ml of basal medium 60 are required for every 1 cm² of gas permeable film 120. When culturing anchorage dependent cells growing in monolayer, advantageously the volume of basal medium 60 exceeds the surface area of gas permeable film 120 by at least a factor of two.

The housing of compartmentalized culture flask 10 can be any biocompatible material. In the preferred embodiment, the housing will provide optical clarity so the medium can be visually monitored for determining the pH of the medium or detecting possible microbial contamination. Polystyrene is a favored selection. Construction of compartmentalized tissue culture flask 10 can be by ultrasonic welding, mechanical fasteners, solvent bonding or any other method which provides leak proof integrity Gas permeable film 120 and membrane 20 can be sealed by o-nngs, gaskets, welding, adhesives, or any other method which provides leak proof integrity. In the preferred embodiment, all materials used in the compartmentalized tissue culture flask 10 should be compatible with gamma sterilization.

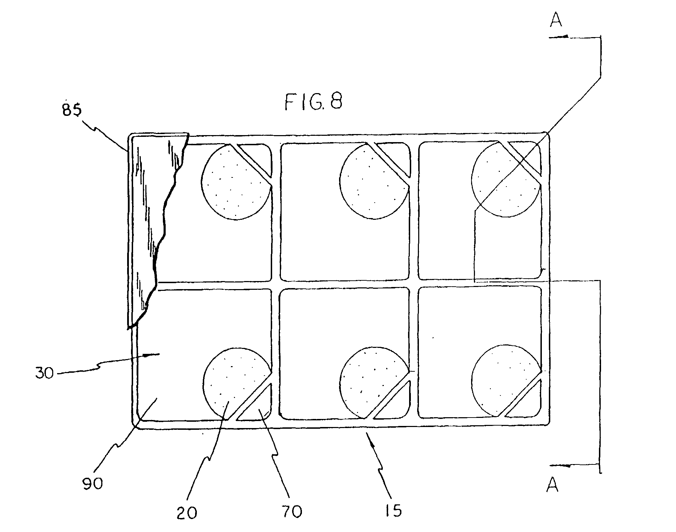

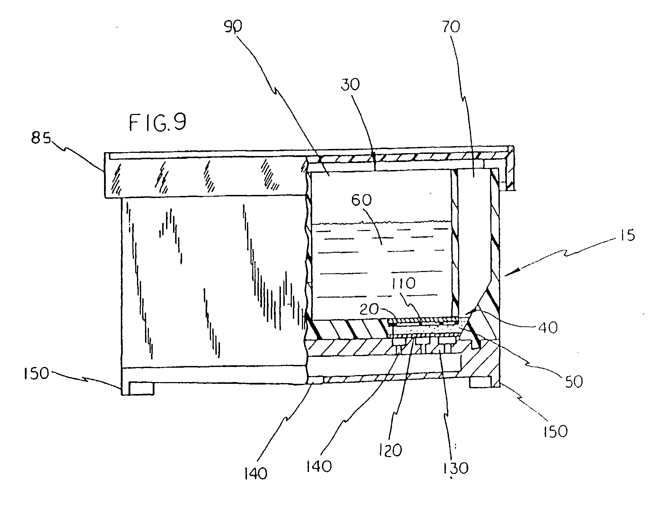

Fig. 8 is a top view of an additional embodiment of this invention in the format of a compartmentalized multiple well tissue culture plate 15. Fig. 9 shows section A-A of Fig. 8. In this configuration, a number of different cultures can conducted in one device. This drawing shows an embodiment that can allow six cultures. The device can constructed to allow more or less cultures if desired. Basal medium 60 resides in basal medium compartment 30. A top cover 85 resides over basal medium compartment 30 and protects basal medium 60 from contaminants.

Fig. 9 is a top view of a further embodiment of a compartmentalized multiple well tissue culture plate 15. In this format basal medium compartment 30 is in communication with more than one cell culture compartment 40.

As described previously in Fig. 2, a configuration where portions of gas permeable film 120 project into cell culture compartment 40 to provide support membrane 20 without the need for membrane support 110 is possible.

As described previously in Fig. 3 , a configuration adapted to include features beneficial for high density cell culture and variable control over the volume of culture medium 50 is possible.

As described previously in Fig. 4 , a configuration that incorporates features for placing the humidified gas of the basal medium compartment 30 in communication with the underside of gas permeable film 120 is possible.

As described previously in Fig. 5 , a compartmentalized multiple well tissue culture plate 15 can be adapted to include skirt 280 to prevent rapid pH changes when the device is removed from a CO₂ incubator, for routine handling, as previously described.

As described previously in Fig. 7 , an embodiment configured to include the features of variable oxygen control compartment 220 is possible.

## Claims

1. A cell culture device comprising a container having
a) a cell culture compartment (40) defined by a lower gas permeable film (120) and an upper sheet (20) selectively permeable to compounds of a predetermined size and impermeable to cells and adapted by appropriate means to allow a culture medium (50) to reside between said upper sheet (20) and said lower gas permeable film (120);
b) a basal medium compartment (30), at least a portion of which resides above said upper sheet (20), and adapted to allow a basal medium (60) to reside upon said upper sheet (20);
c) only a single access port (70) to said cell culture compartment (40);
d) an access port (90) to said basal medium compartment (30); and
e) a gas film support (130) below and in partial contact with said gas permeable film (120) whereby the majority of said gas permeable film (120) is held in a substantially horizontal position such that suspension cells can distribute across the horizontal portion of said gas permeable film (120) and gas transfer into and out of said cell culture compartment (40) is not substantially impaired.

2. A device according to claim 1 wherein a plurality of cell culture compartments (40) are present.

3. A device according to claim 2 wherein a plurality of basal medium compartments (30) are present.

4. A device according to claim 1 wherein said lower gas permeable film (120) includes sections which project into said cell culture compartment (40).

5. A device according to claim 1 wherein an upper membrane support (270) resides above said upper sheet (20) .

6. A device according to claim 1 comprising a container having
f) a gas exchange compartment adapted by appropriate means to provide gaseous communication between a gas within said basal medium compartment (30) and the underside of said lower gas permeable film (120) .

7. A device according to claim 6 wherein a plurality of cell culture compartments.(40) are present.

8. A cell culture device according to claim 1 comprising a container having
f) an oxygen control compartment (220) adapted by appropriate means to provide gaseous communication between the underside of said lower gas permeable film (120) and the ambient environment.

9. A device according to claim 8 wherein said lower gas permeable film (120) includes sections which project into said cell culture compartment.

10. A cell culture device according to claim 8 wherein a plurality of cell culture compartments (40) are present.

11. A cell culture device according to claim 8 wherein said oxygen control compartment is comprised of a further gas permeable film (230) which is disposed in a horizontal position below said lower gas permeable film (120), a separating means between said lower gas permeable film (120) and said further gas permeable film (230) to define said oxygen control compartment (220), said oxygen control compartment (220) being adapted to contain fluid, and an access port whereby liquid can be added to or removed from said oxygen control compartment to control the rate of gas transport.

12. A method of culturing cells comprising the steps:
a) forming a cell culture compartment (40) defined by a lower gas permeable film (120) and an upper sheet (20) selectively permeable to compounds of a predetermined size and impermeable to cells and adapted by appropriate means to allow a culture medium (50) to reside between said upper sheet (20) and said lower gas permeable film (120);
b) forming a basal medium compartment (30), at least a portion of which resides above said upper sheet (20), and adapted to allow a basal medium (60) to reside upon said upper sheet (20);
c) forming only a single access port (70) to said cell culture compartment (40);
d) forming an access port (90) to said basal medium compartment (30);
e) forming a gas film support (130) below and in partial contact with said gas permeable film (120) whereby the majority of said gas permeable film (120) is held in a substantially horizontal position such that suspension cells can distribute across the horizontal portion of said gas permeable film (120) and gas transfer into and out of said cell culture compartment (40) is not substantially impaired;
f) placing a basal medium (60) in said basal medium compartment (30);
g) placing cells and a cell culture medium (50) in said cell culture compartment (40), said cell culture medium (50) being in contact with the underside of said upper sheet (20); and
h) maintaining said cells at a selected temperature.

13. A method according to claim 12 wherein said lower gas permeable film (120) includes sections which project into said cell culture compartment (40).

14. A method according to claim 12 wherein a plurality of said cell culture compartments (40) are formed.

## Patentansprüche

1. Zellkulturvorrichtung umfassend einen Behälter mit
a) einem Zellkulturabteil (40), das von einer unteren gasdurchlässigen Schicht (120) und einem oberen Blatt (20), das selektiv für Verbindungen einer vorgegebenen Größe durchlässig und für Zellen undurchlässig ist, begrenzt wird und durch geeignete Maßnahmen dafür ausgelegt ist, ein Kulturmedium (50) zwischen dem oberen Blatt (20) und der unteren gasdurchlässigen Schicht (120) aufzunehmen;
b) einem Basalmediumabteil (30), von dem sich mindestens ein Teil über dem oberen Blatt (20) befindet, und das so ausgebildet ist, daß ein Basalmedium (60) auf dem oberen Blatt (20) liegen kann;
c) nur einer einzigen Zugangsöffnung (70) zu dem Zellkulturabteil (40);
d) einer Zugangsöffnung (90) zu dem Basalmediumabteil (30); und
e) einen gasdurchlässigen Schichtträger (130), der unterhalb der gasdurchlässigen Schicht (120) angeordnet ist und mit dieser teilweise in Kontakt steht, wodurch der größte Teil der gasdurchlässigen Schicht (120) in einer im wesentlichen horizontalen Lage gehalten wird, so daß sich Zellen einer Suspension entlang des horizontalen Abschnitts der gasdurchlässigen Schicht (120) verteilen können und ein Gasaustausch mit dem Zellkulturabteil (40) nicht wesentlich behindert wird.

2. Vorrichtung nach Anspruch 1, die mehrere Zellkulturabteile (40) aufweist.

3. Vorrichtung nach Anspruch 2, die mehrere Basalmediumabteile (30) aufweist.

4. Vorrichtung nach Anspruch 1, bei der die untere gasdurchlässige Schicht (120) Abschnitte aufweist, die in das Zellkulturabteil (40) hineinragen.

5. Vorrichtung nach Anspruch 1, bei der ein oberer Membranhalter (270) über dem oberen Blatt (20) angeordnet ist.

6. Vorrichtung nach Anspruch 1, umfassend einen Behälter mit
f) einem Gasaustauschfach, das durch geeignete Mittel dafür ausgelegt ist, eine gasdurchlässige Verbindung zwischen einem Gas in dem Basalmediumabteil (30) und der Unterseite der unteren gasdurchlässigen Schicht (120) zu schaffen.

7. Vorrichtung nach Anspruch 6, die mehrere Zellkulturabteile aufweist.

8. Zellkulturvorrichtung nach Anspruch 1, umfassend einen Behälter mit
f) einem Sauerstoffkontrollabteil (220), das durch geeignete Mittel dafür ausgelegt ist, eine gasdurchlässige Verbindung zwischen der Unterseite der unteren gasdurchlässigen Schicht (120) und der umgebenden Umwelt zu schaffen.

9. Vorrichtung nach Anspruch 8, bei der die gasdurchlässige Schicht (120) Abschnitte aufweist, die in das Zellkulturabteil hineinragen.

10. Zellkulturvorrichtung nach Anspruch 8, bei der mehrere Zellkulturabteile (40) vorhanden sind.

11. Zellkulturvorrichtung nach Anspruch 8, bei der das Sauerstoffkontrollabteil von einer weiteren gasdurchlässigen Schicht (230), die in einer horizontalen Lage unter der unteren gasdurchlässigen Schicht (120) angeordnet ist, und einem Trennmittel gebildet wird, das zwischen der unteren gasdurchlässigen Schicht (120) und der weiteren gasdurchlässigen Schicht (230) zum Begrenzen des Sauerstoffkontrollabteils (220) angeordnet ist, wobei das Sauerstoffkontrollabteil (220) dafür ausgelegt ist, Flüssigkeit aufzunehmen, und eine Einlaßöffnung hat, durch die Flüssigkeit eingeleitet oder aus dem Sauerstoffkontrollabteil entfernt werden kann, um die Rate des Gastransports zu steuern.

12. Verfahren zum Züchten von Zellen umfassend die Schritte:
a) Schaffen eines Zellkulturabteils (40), das von einer unteren gasdurchlässigen Schicht (120) und einem oberen Blatt (20), das selektiv für Verbindungen einer vorgegebenen Größe durchlässig und für Zellen undurchlässig ist, begrenzt wird und durch geeignete Maßnahmen dafür ausgelegt ist, ein Kulturmedium (50) zwischen dem oberen Blatt (20) und der unteren gasdurchlässigen Schicht (120) aufzunehmen;
b) Schaffen eines Basalmediumabteils (30), von dem sich mindestens ein Teil über dem oberen Blatt (20) befindet und das so ausgebildet ist, daß ein Basalmedium (60) auf dem oberen Blatt (20) liegen kann;
c) Schaffen von nur einer einzigen Zugangsöffnung (70) zu dem Zellkulturabteil (40);
d) Schaffen einer Zugangsöffnung (90) zu dem Basalmediumabteil (30);
e) Schaffen eines gasdurchlässigen Schichtträgers (130) unter der gasdurchlässigen Schicht (120), der diese teilweise berührt, wodurch der größte Teil der gasdurchlässigen Schicht (120) in einer im wesentlichen horizontalen Lage gehalten wird, so daß sich Zellen einer Suspension entlang des horizontalen Abschnitts der gasdurchlässigen Schicht (120) verteilen können und ein Gasaustausch mit dem Zellkulturabteil (40) nicht wesentlich behindert wird;
f) Einbringen eines Basalmediums (60) in das Basalmediumabteil (30);
g) Einbringen von Zellen und einem Zellkulturmedium (50) in das Zellkulturabteil (40), wobei das Zellkulturmedium (50) mit der Unterseite des oberen Blatts (20) in Kontakt ist; und
h) Pflegen der Zellen bei einer ausgewählten Temperatur.

13. Verfahren nach Anspruch 12, bei dem die untere gasdurchlässige Schicht (120) Abschnitte aufweist, die in das Zellkulturabteil (40) hineinragen.

14. Verfahren nach Anspruch 12, bei dem mehrere Zellkulturabteile (40) gebildet werden.

## Revendications

1. Dispositif de culture cellulaire comprenant un conteneur ayant :
a) un compartiment de culture cellulaire (40) défini par un film inférieur perméable au gaz (120) et une feuille supérieure (20) perméable sélectivement aux composés d'une taille prédéterminée et imperméable aux cellules et adapté par des moyens appropriés pour permettre à un milieu de culture (50) de résider entre ladite feuille supérieure (20) et ledit film inférieur perméable au gaz (120) ;
b) un compartiment pour milieu de base (30), dont au moins une portion réside au-dessus de ladite feuille supérieure (20) et adapté pour permettre à un milieu de base (60) de résider sur ladite feuille supérieure (20) ;
c) uniquement un seul orifice d'accès (70) audit compartiment de culture cellulaire (40) ;
d) un orifice d'accès (90) audit compartiment pour milieu de base (30) ; et
e) un support du film dé gaz (130) en dessous et en contact partiel avec ledit film perméable au gaz (120) grâce à quoi la majorité dudit film perméable au gaz (120) est maintenue dans une position essentiellement horizontale de sorte que les cellules en suspension peuvent se répartir sur toute la portion horizontale dudit film perméable au gaz (120) et qu'il n'y a pas de détérioration substantielle du transfert de gaz à destination ou en provenance du compartiment de culture cellulaire (40).

2. Dispositif selon la revendication 1, dans lequel une pluralité de compartiments de cellules (40) sont présents.

3. Dispositif selon la revendication 2, dans lequel une pluralité de compartiments pour milieu de base (30) sont présents.

4. Dispositif selon la revendication 1, dans lequel ledit film inférieur perméable au gaz (120) comprend des sections qui font saillie dans ledit compartiment de culture cellulaire (40).

5. Dispositif selon la revendication 1, dans lequel un support de membrane supérieur (270) réside au-dessus de ladite feuille supérieure (20).

6. Dispositif selon la revendication 1, comprenant un conteneur ayant :
f) un compartiment d'échanges gazeux adapté par des moyens appropriés à la fourniture d'une communication gazeuse entre un gaz se trouvant à l'intérieur dudit compartiment supérieur pour milieu de base (30) et le dessous dudit film inférieur perméable au gaz (120).

7. Dispositif selon la revendication 6, dans lequel une pluralité de compartiments de culture cellulaire (40) sont présents.

8. Dispositif de culture cellulaire selon la revendication 1, comprenant un conteneur ayant :
f) un compartiment de contrôle d'oxygène (220) adapté par des moyens appropriés de manière à assurer une communication gazeuse entre le dessous dudit film inférieur perméable au gaz (120) et l'environnement ambiant.

9. Dispositif selon la revendication 8, dans lequel ledit film inférieur perméable au gaz (120) comprend des sections qui font saillie dans ledit compartiment de culture cellulaire.

10. Dispositif de culture cellulaire selon la revendication 8, dans lequel une pluralité de compartiments de culture cellulaire (40) sont présents.

11. Dispositif de culture cellulaire selon la revendication 8, dans lequel ledit compartiment de contrôle d'oxygène comporte un autre film perméable au gaz (230) qui est disposé dans une position horizontale sous ledit film inférieur perméable au gaz (120), des moyens de séparation entre ledit film inférieur perméable au gaz (120) et ledit autre film perméable au gaz (230) afin de définir ledit compartiment de contrôle d'oxygène (220), ledit compartiment de contrôle d'oxygène (220) étant adapté pour contenir un fluide, et un orifice d'accès par lequel il est possible d'ajouter du liquide audit compartiment de contrôle de l'oxygène ou d'en retirer afin de contrôler le taux de transport gazeux.

12. Procédé de culture de cellules comprenant les étapes consistant à :
a) former un compartiment de culture cellulaire (40) défini par un film inférieur perméable au gaz (120) et une feuille supérieure (20) sélectivement perméable aux composés d'une taille prédéterminée et imperméable aux cellules et adapté par des moyens appropriés pour permettre à un milieu de culture (50) de résider entre ladite feuille supérieure (20) et ledit film inférieur perméable au gaz (120) ;
b) former un compartiment pour milieu de base (30), dont au moins une portion réside au-dessus de ladite feuille supérieure (20), et adapté pour permettre à un milieu de base (60) de résider sur ladite feuille supérieure (20) ;
c) former uniquement un seul orifice d'accès (70) audit compartiment de culture cellulaire (40) ;
d) former un orifice d'accès (90) audit compartiment pour milieu de base (30) ;
e) former un support du film de gaz (130) en dessous et en contact partiel avec ledit film perméable au gaz (120), grâce à quoi la majorité dudit film perméable au gaz (120) est maintenue dans une position essentiellement horizontale de sorte que les cellules en suspension peuvent se répartir sur toute la portion horizontale dudit film perméable au gaz (120) et qu'il n'y a pas de détérioration appréciable du transfert de gaz à destination ou en provenance du compartiment de culture cellulaire (40) ;
f) placer un milieu de base (60) dans ledit compartiment pour milieu de base (30) ;
g) placer des cellules et un milieu de culture cellulaire (50) dans ledit compartiment de culture cellulaire (40), ledit milieu de culture cellulaire (50) étant en contact avec le dessous de ladite feuille supérieure (20) ; et
h) maintenir lesdites cellules à une température choisie.

13. Procédé selon la revendication 12, dans lequel ledit film inférieur perméable au gaz (120) comprend des sections qui font saillie dans ledit compartiment de culture cellulaire (40).

14. Procédé selon la revendication 12, dans lequel une pluralité desdits compartiments de culture cellulaire (40) est formée.
